(19) Europäisches Patentamt
European Patent Office
Office européen des brevets

(11) Veröffentlichungsnummer: **0 090 305**
**B1**

(12) **EUROPÄISCHE PATENTSCHRIFT**

(45) Veröffentlichungstag der Patentschrift: **22.04.87**

(21) Anmeldenummer: **83102763.6**

(22) Anmeldetag: **21.03.83**

(51) Int. Cl.⁴: **C 09 B 57/02**, C 07 D 491/044, D 06 P 1/16, C 08 K 5/34, D 06 F 1/06, C 09 K 3/00

# (54) Neue Azacumarine, Verfahren zu ihrer Herstellung und ihre Verwendung als Farbstoffe.

(30) Priorität: **24.03.82 DE 3210776**

(43) Veröffentlichungstag der Anmeldung: **05.10.83 Patentblatt 83/40**

(45) Bekanntmachung des Hinweises auf die Patenterteilung: **22.04.87 Patentblatt 87/17**

(84) Benannte Vertragsstaaten: **CH DE FR GB LI**

(56) Entgegenhaltungen:
**EP - A - 0 004 322**
**FR - A - 1 228 637**
**FR - A - 2 353 615**
**GB - A - 2 006 253**
**US - A - 4 200 753**

**LIEBIGS ANNALEN DER CHEMIE, Mai 1981, Seiten 811-818, Verlag Chemie GmbH, Weinheim, DE; O.S. WOLFBEIS *et al.*: "2-Substituierte Pyrano[2,3-c]isochinolin-3,6-dione und Merocyaninfarbstoffe aus Homophthalsäureimiden"**

(73) Patentinhaber: **HOECHST AKTIENGESELLSCHAFT, Postfach 80 03 20, D-6230 Frankfurt am Main 80 (DE)**

(72) Erfinder: **Deger, Hans-Mathias, Dr., Am Rheingauer Weg 6, D-6238 Hofheim am Taunus (DE)**
Erfinder: **Konz, Elmar, Dr., Brüningstrasse 9, D-6233 Kelkheim (Taunus) (DE)**

**EP 0 090 305 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind neue Azacumarine der allgemeinen Formel

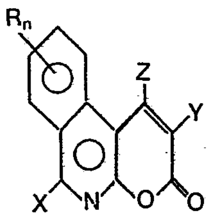

(1)

in welcher X einen ggfs. beispielsweise durch ein oder mehrere Fluor-, Chlor- oder Bromatome oder eine oder mehrere Alkyl$_{C_1-C_4}$, Alkoxy$_{C_1-C_4}$, Cyan-, Nitro-, Amino-, Monoalkyl$_{C_1-C_4}$amino-, Dialkyl$_{C_1-C_4}$amino-, Monoarylamino-, Diarylamino-, Carboxyl-, Carbonamid-, Carbonmonoalkyl$_{C_1-C_4}$amid-, Carbondialkyl$_{C_1-C_4}$amid-, Alkoxy$_{C_1-C_4}$carbonyl- oder Trifluormethylgruppen substituierten Phenylrest oder einen ggfs. beispielsweise durch Chlor- oder Bromatome oder Alkyl$_{C_1-C_4}$ oder Nitrogruppen substituierten Thiophen-, Pyridin-, Naphthalin- oder Pyrenrest bedeutet, Y ein Wasserstoffatom oder eine Cyan- oder Alkoxy$_{C_1-C_4}$carbonylgruppe, einen ggfs. beispielsweise durch Fluor-, Chlor- oder Bromatome oder Alkyl$_{C_1-C_4}$, Alkoxy$_{C_1-C_4}$, Nitro-, Amino-, Monoalkyl$_{C_1-C_4}$amino- oder Dialkyl$_{C_1-C_4}$aminogruppen substituierten Phenylrest oder einen ggfs. beispielsweise durch Fluor-, Chlor- oder Bromatome oder Alkyl$_{C_1-C_4}$, Alkoxy$_{C_1-C_4}$, Nitro-, Cyan- oder Carbonsäurealkyl$_{C_1-C_4}$estergruppen substituierten Thiophen-, Benzothiophen-, Furan-, Benzofuran-, Imidazol-, Benzimidazol-, Thiazol-, Benzthiazol-, Oxazol- oder Benzoxazolrest darstellt, Z ein Wasserstoffatom oder eine Carbonsäureamid- oder Cyangruppe bedeutet, R ein Wasserstoff-, Fluor-, Chlor- oder Bromatom oder eine Alkyl$_{C_1-C_4}$, Alkoxy$_{C_1-C_4}$, Nitro-, Amino-, Monoalkyl$_{C_1-C_4}$amino-, Dialkyl$_{C_1-C_4}$amino- oder Cyangruppe darstellt und n für die Zahl 1 oder 2 steht, sowie ein Verfahren zu ihrer Herstellung und ihre Verwendung als Farbstoffe zum Färben oder Bedrucken von synthetischen Fasermaterialien, wie beispielsweise solchen aus Cellulosedi-, $-2\frac{1}{2}-$ oder -triacetat, Polyamiden, Polyurethanen, Polycarbonaten oder insbesondere linearen Polyestern, die ggfs. in Mischung mit natürlichen Fasermaterialien, wie solchen aus Cellulosefasern oder Wolle, vorliegen, zum Färben von Kunststoffen in der Masse, sowie zum Schmelzspinnfärben von Polyestern, insbesondere von Polyäthylenterephthalat, sowie ihre Verwendung in Lichtsammelsystemen.

Die Farbstoffe der weiter oben genannten allgemeinen Formel (1) können hergestellt werden, indem man eine Verbindung der allgemeinen Formel (2)

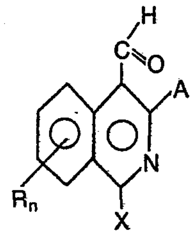

(2)

in welcher X, R und n die vorstehend genannten Bedeutungen haben, und A ein Chlor- oder Bromatom darstellt, mit einer Verbindung der allgemeinen Formel (3)

$$B-CH_2-Y \qquad (3)$$

in welcher Y die vorstehend genannte Bedeutung hat, und B eine Cyano- oder Alkoxy$_{C_1-C_4}$carbonylgruppe darstellt, in an sich bekannter Weise kondensiert, die hierbei erhaltene Verbindung der allgemeinen Formel (4)

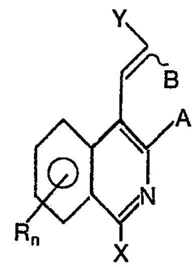

(4)

in welcher A, B, X, Y, R und n die angegebenen Bedeutungen haben, mit einer nichtoxydierenden wässrigen Mineralsäure, vorzugsweise Schwefelsäure oder Phosphorsäure einer Konzentration von 600–900 g/kg, vorzugsweise 700–850 g/kg, bei Temperaturen von 80 bis 140 °C, vorzugsweise 100–120 °C, cyclisiert zu einer Verbindung der genannten allgemeinen Formel (1) mit Z=H und diese ggfs. nach an sich bekannten Verfahren [DE-OS 28 44 299] mit einem Cyanidsalz umsetzt und die hierbei als Zwischenprodukt gebildete Anlagerungsverbindung gleichzeitig oder anschliessend nach an sich bekannten Verfahren mit einem Oxydationsmittel behandelt unter Erhalt der Verbindung der allgemeinen Formel (1) mit Z = CN und diese ggfs. in an sich bekannter Weise verseift zur Verbindung der allgemeinen Formel (1) mit Z = CO–NH$_2$.

Die Verbindungen der weiter oben genannten allgemeinen Formel (2) können auf die in der Europäischen Offenlegungsschrift 4322 beschriebene Weise hergestellt werden, indem man 1,4-Dihydro-3(2H)-isochinolinderivate mit einem Vilsmeier-Haack-Komplex bei Temperaturen zwischen vorzugsweise 0 und 50 °C umsetzt.

Die Kondensation der Aldehyde der genannten allgemeinen Formel (2) mit den Verbindungen der genannten allgemeinen Formel (3) erfolgt entweder unter den Bedingungen der Knoevenagel-Kondensation oder durch Umsetzung in polaren Lösemitteln unter Katalyse mit starken organischen oder anorganischen Basen. Als Lösemittel eignen sich hierbei niedere aliphatische Alkohole,

wie Methanol, Äthanol oder Propanol sowie stickstoffhaltige Lösemittel, wie Pyridin, Picolin, N,N-Dimethylformamid oder N-Methylpyrrolidon. Als Basen eignen sich Alkalimetallhydroxide, wie Kaliumhydroxyd oder Natriumhydroxyd, ferner Alkalimetallalkoholate, wie Natriummethanolat, Kaliummethanolat, Natriumäthanolat oder Kaliumäthanolat, sowie Stickstoffbasen, wie Piperidin, Pyrrolidon oder N-Methylanilin. Die Umsetzung erfolgt bei Temperaturen von 0 bis 80 °C, vorzugsweise 20–60 °C.

Die Knoevenagel-Reaktion wird durchgeführt in vorzugsweise unpolaren Lösemitteln in Gegenwart anorganischer oder organischer Kondensationsmittel bei Temperaturen von 50–150 °C, vorzugsweise 80–120 °C unter gleichzeitiger Entfernung des Reaktionswassers. Als Lösemittel eignen sich Kohlenwasserstoffe, wie Benzol, Toluol, Xylol oder Cyclohexan, chlorierte Kohlenwasserstoffe, wie Chlorbenzol oder Chloroform, oder Ester organischer Säuren, wie Methylacetat oder Äthylacetat. Als Kondensationsmittel sind Stickstoffbasen, wie Piperidin, Pyrrolidin oder N-Methylanilin, sowie Salze von Stickstoffbasen, wie Piperidin- und Ammoniumacetat, geeignet.

Die Verbindung der genannten Formel (1) mit Z = CN kann hergestellt werden, indem man die Verbindung der allgemeinen Formel (1) mit Z = H nach an sich bekannten Verfahren, wie sie beispielsweise in der DE-OS 28 44 299 niedergelegt sind, in einem Lösemittel mit einem Cyanidsalz umsetzt, und die hierbei als Zwischenprodukte gebildete Anlagerungsverbindung gleichzeitig oder nachträglich mit Oxidationsmitteln behandelt.

Als Cyanidsalze verwendet man hierbei vorzugsweise wasserlösliche Alkalicyanide, wie Natrium- oder Kaliumcyanid, wahlweise als Feststoff oder insbesondere in Form von konzentrierten wässrigen Lösungen.

Geeignete Lösemittel sind polare organische Lösemittel wie Alkohole, beispielsweise Methanol, Äthanol, Isopropanol oder Äthylenglykol, insbesondere aber aprotische dipolare Lösemittel, wie Dimethylformamid, Pyridin oder Picolin.

Die Umsetzung mit den Cyanidsalzen wird im Temperaturbereich von 0–100 °C, vorzugsweise 15–70 °C, und die Oxidation der als Zwischenprodukt angefallenen Anlagerungsverbindung im Bereich von −10 bis +30 °C, vorzugsweise bei −5 bis +10 °C durchgeführt.

Zur Oxidation der als Zwischenprodukt anfallenden Anlagerungsverbindung eignen sich Oxidationsmittel, wie Wasserstoffperoxide, Persulfate oder organische Peroxide, wie beispielsweise Di-benzoylperoxid, ferner Salpetersäure, Brom oder Bleitetracetat.

Die neuen Azacumarine der genannten Formel (1) stellen gelbe bis schwarze kristalline Pulver dar, die sich in organischen Lösemitteln, wie Alkoholen, Estern, Amiden, Äthern, Ketonen oder chlorierten Kohlenwasserstoffen, mit intensiver grüngelber bis tief roter Fluoreszenz lösen.

Die neuen Cumarinverbindungen eignen sich allein oder in Mischungen untereinander oder mit anderen Farbstoffen, vorzugsweise in zubereiteter Form, wie beispielsweise in wässriger Dispersion oder in Lösung organischer Lösemittel oder in Emulsion oder Dispersion, die neben einem Lösemittel oder einem Lösemittelgemisch noch Wasser enthalten können, zum Färben oder Bedrucken von synthetischen Fasermaterialien, wie beispielsweise solchen aus Cellulose-di-,

$$-2\frac{1}{2}-$$

oder -triacetat, Polyamiden, wie Poly-ε-caprolactam oder Poly-hexamethylen-diaminadipat, ferner solchen aus Polyurethanen oder Polycarbonaten, insbesondere aber solchen aus linearen Polyestern, wie Polyäthylenterephthalaten, die jeweils in Mischung mit natürlichen Fasermaterialien, wie solchen aus Cellulosefasern oder Wolle, vorliegen können. Die genannten Fasermaterialien können hierbei in verschiedenen Verarbeitungszuständen, beispielsweise als Kammzug, Flocke, Fäden, Gewebe oder Gewirke, vorliegen.

Die Applikation der erfindungsgemässen Farbstoffe erfolgt in prinzipiell bekannter Weise in der Regel aus wässriger Dispersion; sie kann jedoch auch aus organischen Lösemitteln vorgenommen werden. Die Dispergierung der Farbstoffe kann beispielsweise durch Mahlen in Gegenwart eines Dispergiermittels, wie beispielsweise des Kondensationsproduktes aus Formaldehyd und technischem m-Kresol und Natriumbisulfit, das mit Schwefelsäure neutral gestellt wurde, vorgenommen werden. Im übrigen richten sich die Färbebedingungen weitgehend nach der Art der vorliegenden synthetischen Fasermaterialien und deren Verarbeitungszustand.

Beispielsweise erfolgt das Färben von geformten Gebilden aus Celluloseacetat in einem Temperaturbereich von 75 bis 85 °C. Cellulosetriacetatfasern werden bei Temperaturen zwischen etwa 90 und 125 °C gefärbt. Das Aufbringen der Farbstoffe auf Polyamidfasermaterialien erfolgt im Temperaturbereich zwischen etwa 90 und 120 °C. Für das Färben von Fasermaterialien aus Polyestern benutzt man die hierfür bekannten Methoden, indem man das Fasermaterial in Gegenwart von Carriern, wie o- oder p-Phenylphenol, Methylnaphthalin oder Methylsalicylat, bei Temperaturen von 100 °C bis etwa 130 °C oder aber ohne Anwendung von Carriern bei entsprechend höheren Temperaturen, beispielsweise zwischen 120 und 140 °C färbt. Ausserdem kann man auch so verfahren, dass man die Farbstoffe durch Klotzen, mit oder ohne Verdickungsmittel, beispielsweise Tragant-Verdickung, aufbringt und durch Hitzeeinwirkung, beispielsweise durch Dampf oder Trockenhitze während etwa $^1/_2$ bis 30 Minuten bei Temperaturen im Bereich von etwa 100 bis 230 °C fixiert. Das so gefärbte Material wird dann zur Verbesserung der Reibechtheit zweckmässig von oberflächlich anhaftendem Farbstoff befreit, beispielsweise durch Spülen oder eine reduktive Nachbehandlung. Diese Nachbehandlung erfolgt im allgemeinen bei 60 bis 120 °C in einer wässrigen Natronlauge, Natriumdithionit und ein nichtionogenes Waschmittel, wie beispielsweise ein Äthylenoxid-Phenol-Additionsprodukt, enthaltenden Flotte.

Zur Färbung der synthetischen Fasermaterialien aus organischen Lösemitteln kann man beispielsweise so verfahren, dass man bei Raumtemperatur oder darüber, vorzugsweise bei 70 bis 130 °C, gegebenenfalls unter Druck, den Farbstoff aus der Lösung auf die Faser aufziehen lässt oder so, dass man in einer kontinuierlichen Arbeitsweise Gewebe oder Gewirke mit einer Farbstofflösung imprägniert, trocknet und einer kurzzeitigen Hitzeeinwirkung, beispielsweise bei Temperaturen von 180 bis 210 °C, unterwirft. Als Lösemittel für das Ausziehverfahren seien beispielsweise mit Wasser nicht mischbare Lösemittel mit Siedepunkten zwischen 40 und 170 °C genannt, wie etwa die aliphatischen Halogenkohlenwasserstoffe, wie Methylenchlorid, Trichloräthan, Trichloräthylen oder Trifluortrichloräthan. Insbesondere für ein kontinuierliches Färbeverfahren kommen auch mit Wasser mischbare Lösemittel, wie beispielsweise Alkohole oder Dimethylformamid, in Betracht. Die Lösemittel können natürlich auch als Mischungen vorliegen und weitere in Lösemitteln lösliche Hilfsmittel, wie beispielsweise Oxalkylierungsprodukte von Fettalkoholen, Alkylphenolen oder Fettsäuren, enthalten.

Zur Herstellung von Drucken auf den synthetischen Fasermaterialien, beispielsweise solchen aus Polyestern, Polyamiden oder Cellulosetriacetat, können die Farbstoffe in Form wasserhaltiger Zubereitungen angewandt werden, die neben dem fein verteilten Farbstoff geeignete Verdickungsmittel enthalten können. Die Fixierung erfolgt beispielsweise nach dem Drucken und Trocknen durch Dämpfen bei Atmosphärendruck oder unter erhöhtem Druck bis zu 2,5 atü während 10 bis 60 Minuten. Ebenso kann man die Fixierung durch die Einwirkung von Heissluft von 160 bis 120 °C während 30 Sekunden bis 10 Minuten bewirken.

Zusätzlich eignen sich die erfindungsgemässen Farbstoffe zum Färben von Kunststoffen in der Masse, beispielsweise von Polyvinylchlorid, Polyolefinen oder Polystyrol, sowie zum Schmelzspinnfärben von Polyestern, beispielsweise von Polyäthylenterephthalat.

Mit den neuen Azacumarinen lassen sich die genannten Fasermaterialien zum Teil extrem brillant grünstichig gelb bis marineblau färben. Die Färbungen zeichnen sich durch hohe Farbstärke, gutes Aufbau- und Ziehvermögen, sowie durch gute Gesamtechtheiten aus.

Die neuen Cumarinverbindungen eignen sich ferner auch zur Verwendung in Lichtsammelsystemen, und zwar sowohl in fester Matrix als auch gelöst in einem Lösemittel. Solche Lichtsammelsysteme werden in Verbindung mit Photoelementen als Solarzellen und in fluoreszenzaktivierten Anzeigeelementen eingesetzt.

Die in den nachstehenden Beispielen angegebenen Teile bedeuten Gewichtsteile.

Beispiel 1

88,2 Teile 1-Phenyl-3-chlor-4-formylisochinolin werden mit 37,2 Teilen 2-Cyanmethyl-5,6-dimethyl-benzoxazol und 3 Teilen Ammoniumacetat in 350 Teilen Benzol $2^1/_2$ Stunden am Wasserabscheider zum Rückfluss erhitzt. Das entstandene Reaktionsprodukt wird bei 5 °C abgesaugt, getrocknet und in 300 Teilen 80%iger Schwefelsäure 2 Stunden bei 120 °C gerührt. Das erkaltete Reaktionsgemisch wird unter Rühren auf 1000 Teile Eis gegossen, mit 30 gewichtsprozentiger Natronlauge auf pH 6 gestellt, abgesaugt, mit Wasser sulfatfrei gewaschen und bei 100 °C im Vakuum bis zur Gewichtskonstanz getrocknet (71 Teile). Nach dem Umkristallisieren aus N,N-Dimethylformamid erhält man 57,9 Teile (71% der Theorie) einer Verbindung der Formel

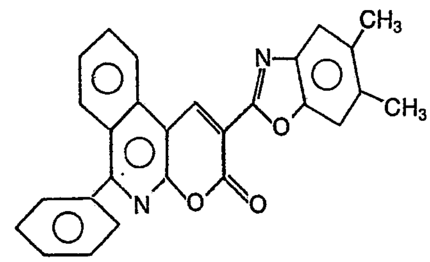

in Form gelber Kristalle vom Schmelzpunkt >300 °C, die Polyesterfasermaterialien in lebhafter, grüngelber Nuance färben.

Beispiel 2

33,7 Teile 1-(4-Diäthylaminophenyl)-3-chlor-4-formylisochinolin werden mit 17,4 Teilen 2-Cyanmethylbenzthiazol und 1 Teil Piperidinacetat in 150 Teilen Toluol 1 Stunde am Wasserabscheider zum Rückfluss erhitzt. Das entstandene Kondensationsprodukt wird bei 5 °C abgesaugt, getrocknet und in 100 Teilen 80%iger Phosphorsäure 2 Stunden bei 120 °C cyclisiert. Das erkaltete Reaktionsgemisch wird unter Rühren auf 300 Teile Eis gegossen, mit 30 gewichtsprozentiger Natronlauge auf pH 6–7 gestellt, abgesaugt, mit Wasser sulfatfrei gewaschen und bei 100 °C im Vakuum bis zur Gewichtskonstanz getrocknet (40 Teile). Nach dem Umkristallisieren aus N,N-Dimethylformamid erhält man 30 Teile (63% der Theorie) einer Verbindung der Formel

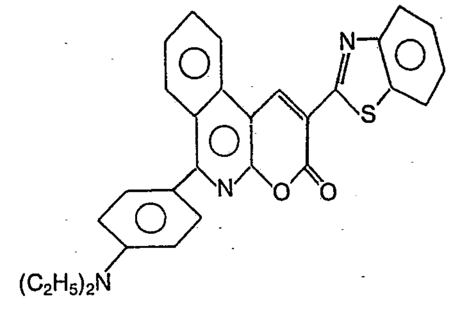

in Form schwarzer, metallisch glänzender Nadeln vom Schmelzpunkt 258–260 °C, die Polyesterfasermaterialien in reiner, lebhaft fluoreszierender roter Nuance färben.

Beispiel 3

26,7 Teile 1-Phenyl-3-chlor-4-formylisochinolin und 17,7 Teile Benzylcyanid werden mit 46 Teilen einer 30gewichtsprozentigen Natriummethanolatlösung in 250 Teilen Methanol 24 Stunden bei Raumtemperatur gerührt. Das Reaktionsgemisch wird auf 1000 Teile Wasser gegossen, mit 2 n HCl auf pH 3 gestellt, abgesaugt, mit Wasser chloridfrei gewaschen und bei 100 °C im Vakuum bis zur Gewichtskonstanz getrocknet. Das Kondensationsprodukt wird in 200 Teilen 80%iger Schwefelsäure 6 Stunden bei 120 °C cyclisiert, auf 1000 Teile Wasser gegossen, abgesaugt, mit Wasser sulfatfrei gewaschen und bei 100 °C im Vakuum bis zur Gewichtskonstanz getrocknet (30 Teile). Nach dem Umkristallisieren aus Dioxan erhält man 18 Teile (51% der Theorie) der Verbindung der Formel

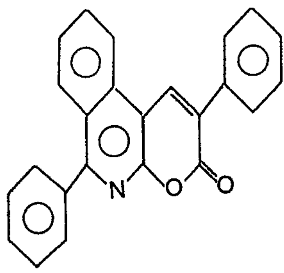

in Form gelber Kristalle vom Schmelzpunkt 257–258 °C, die Polyesterfasermaterialien grün fluoreszierend färbt.

Verfährt man analog den vorstehenden Beispielen 1–3, so erhält man weitere erfindungsgemässe Farbstoffe, die in der nachstehenden Tabelle aufgeführt sind:

Allgemeine Formel der Farbstoffe der nachstehenden Tabellenbeispiele 4–58:

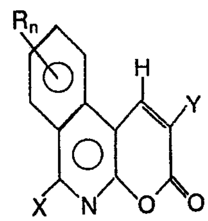

| Beispiel Nr. | X | Y | R | n | Farbton auf Polyesterfasern |
|---|---|---|---|---|---|
| 4 | 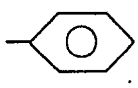 | 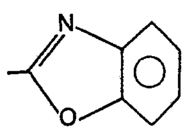 | H | – | gelbgrün |
| 5 | 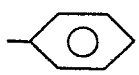 | 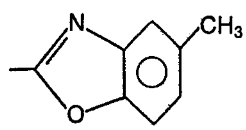 | H | – | gelbgrün |
| 6 | 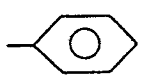 | 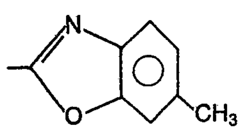 | H | – | gelbgrün |
| 7 | 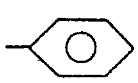 | 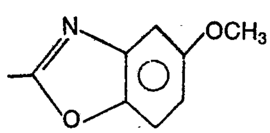 | H | – | gelbgrün |
| 8 | 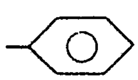 | 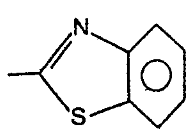 | H | – | gelbgrün |
| 9 | 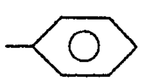 | 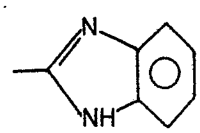 | H | – | gelbgrün |

| Beispiel Nr. | X | Y | R | n | Farbton auf Polyesterfasern |
|---|---|---|---|---|---|
| 10 | 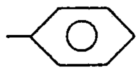 | 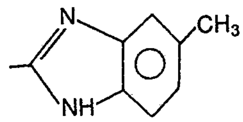 | H | – | gelbgrün |
| 11 | 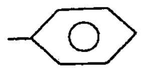 | 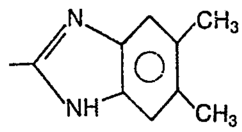 | H | – | gelbgrün |
| 12 | 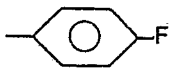 | 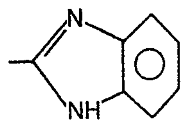 | H | – | gelbgrün |
| 13 | 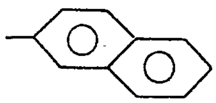 | 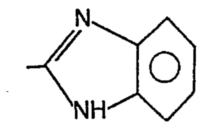 | H | – | gelbgrün |
| 14 | 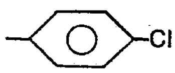 | 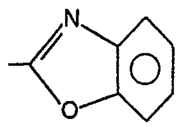 | H | – | gelbgrün |
| 15 | 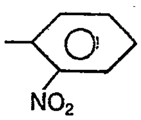 | 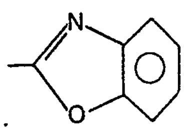 | H | – | gelbgrün |
| 16 | 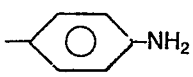 | 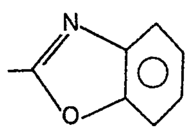 | H | – | rot |
| 17 | 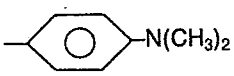 | 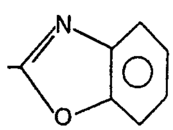 | H | – | rot |
| 18 | 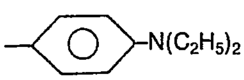 | 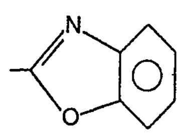 | H | – | rot |
| 19 | 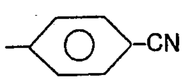 | 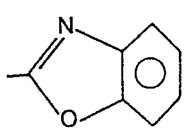 | H | – | gelbgrün |
| 20 | 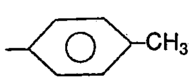 | 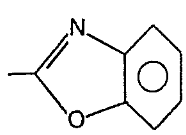 | H | – | gelbgrün |

| Beispiel Nr. | X | Y | R | n | Farbton auf Polyesterfasern |
|---|---|---|---|---|---|
| 21 | 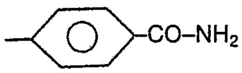 | 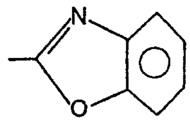 | H | – | gelbgrün |
| 22 | 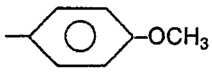 | 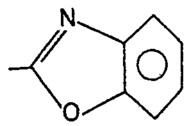 | H | – | brillantgelb |
| 23 | 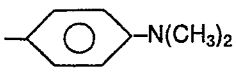 | 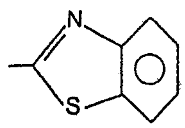 | H | – | rot |
| 24 | 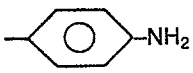 | 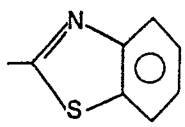 | H | – | rot |
| 25 | 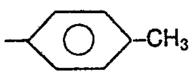 | 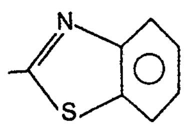 | H | – | gelbgrün |
| 26 | 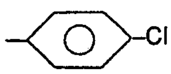 | 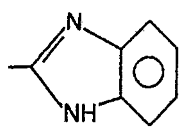 | H | – | gelbgrün |
| 27 | 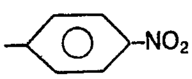 | 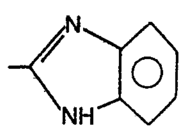 | H | – | gelbgrün |
| 28 | 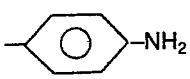 | 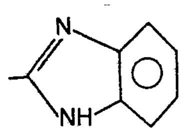 | H | – | gelbstichigrot |
| 29 | 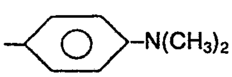 | 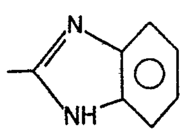 | H | – | rot |
| 30 | 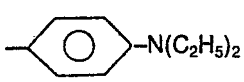 | 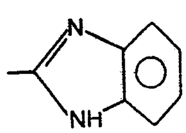 | H | – | rot |
| 31 | 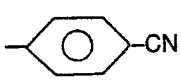 | 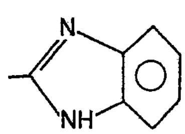 | H | – | gelbgrün |
| 32 | 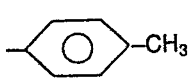 | 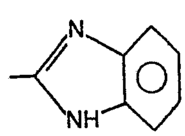 | H | – | gelbgrün |

| Beispiel Nr. | X | Y | R | n | Farbton auf Polyesterfasern |
|---|---|---|---|---|---|
| 33 | 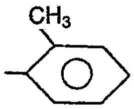 | 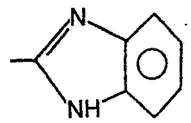 | H | – | gelbgrün |
| 34 | 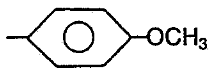 | 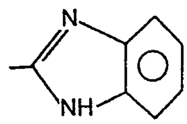 | H | – | brillantgelb |
| 35 | 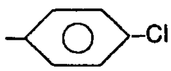 | 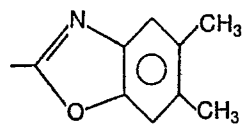 | H | – | gelbgrün |
| 36 | 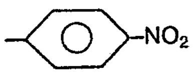 | 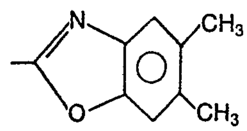 | H | – | gelbgrün |
| 37 | 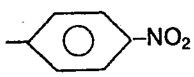 | 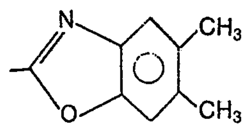 | H | – | gelbgrün |
| 38 | 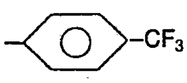 | 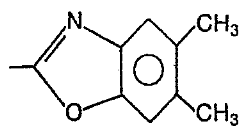 | H | – | gelbgrün |
| 39 | 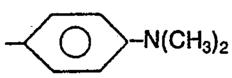 | 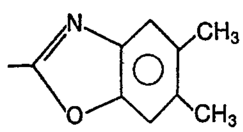 | H | – | gelbgrün |
| 40 | 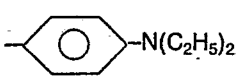 | 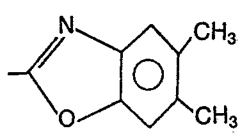 | H | – | gelbgrün |
| 41 | 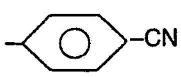 | 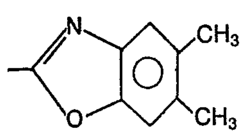 | H | – | gelbgrün |
| 42 | 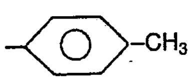 | 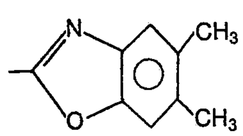 | H | – | gelbgrün |
| 43 | 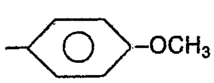 | 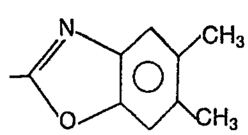 | H | – | brillantgelb |
| 44 | 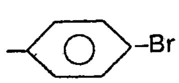 | 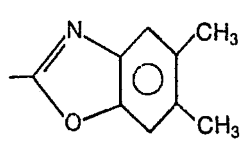 | H | – | gelbgrün |

| Beispiel Nr. | X | Y | R | n | Farbton auf Polyesterfasern |
|---|---|---|---|---|---|
| 45 | 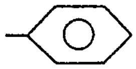 | 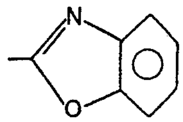 | Cl | 1 | gelbgrün |
| 46 | 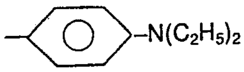 | 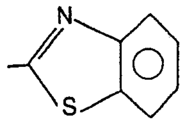 | $NO_2$ | 1 | rot |
| 47 | 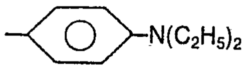 | 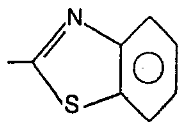 | $OCH_3$ | 2 | rot |
| 48 | 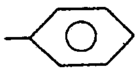 | 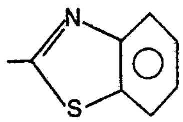 | $OCH_3$ | 2 | gelbgrün |
| 49 | 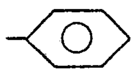 | 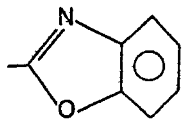 | $CH_3$ | 1 | grünstichiggelb |
| 50 | 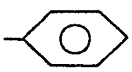 | 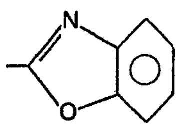 | F | 1 | grünstichiggelb |
| 51 | 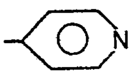 | 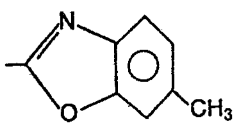 | $NH_2$ | 1 | rot |
| 52 | 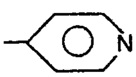 | 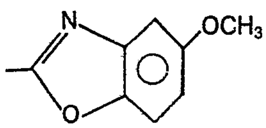 | H | – | grünstichiggelb |
| 53 | 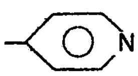 | 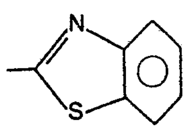 | H | – | grünstichiggelb |
| 54 | 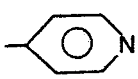 | 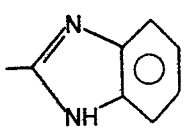 | H | – | grünstichiggelb |
| 55 | 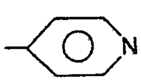 | 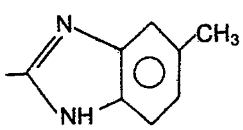 | H | – | grünstichiggelb |
| 56 | 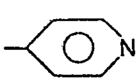 | 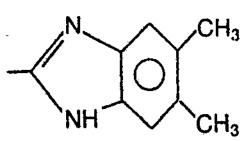 | H | – | grünstichiggelb |

| Beispiel Nr. | X | Y | R | n | Farbton auf Polyesterfasern |
|---|---|---|---|---|---|
| 57 | 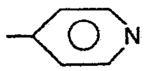 | 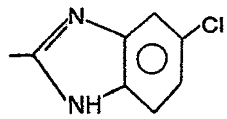 | H | – | grünstichiggelb |
| 58 | 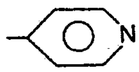 | 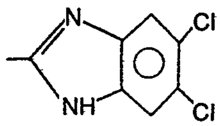 | H | – | grünstichiggelb |

Beispiel 59

41,8 Teile des nach Beispiel 1 hergestellten Farbstoffes werden in 500 Teilen Dimethylformamid suspendiert und mit einer Lösung von 13,2 Teilen Kaliumcyanid in 40 Teilen Wasser bei 0–5 °C tropfenweise versetzt. Man rührt 5 Stunden bei Raumtemperatur nach und tropft anschliessend bei 0–5 °C 17,6 Teile Brom zu. Das Reaktionsprodukt wird nach 5-stündigem Stehen abgesaugt, mit Wasser bromidfrei gewaschen und bei 80 °C im Vakuum getrocknet. Nach dem Umkristallisieren aus Chlorbenzol erhält man 30,1 Teile (68% der Theorie) eines Produktes der Formel

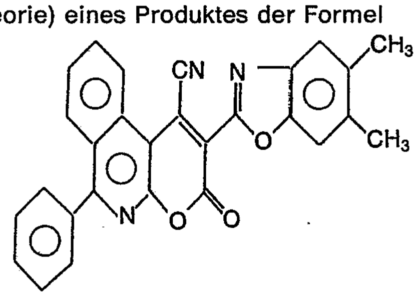

in Form hellroter Kristalle mit dem Schmelzpunkt 264 bis 266 °C, die Polyesterfasern in rotstichig gelben Tönen färben.

Beispiel 60

47,7 Teile des nach Beispiel 2 hergestellten Farbstoffes werden in 350 Teilen Dimethylformamid suspendiert und mit einer Lösung von 13,2 Teilen Kaliumcyanid in 40 Teilen Wasser bei 0–5 °C tropfenweise versetzt. Man rührt 10 Stunden unter Inertgas bei Raumtemperatur nach und tropft anschliessend 17,6 Teile Brom bei Temperaturen von −5 bis +5 °C zu. Das Produkt wird nach 5 stündigem Stehen abgesaugt, mit Wasser bromidfrei gewaschen und bei 80 °C im Vakuum getrocknet. Nach dem Umkristallisieren aus Dimethylformamid erhält man 26,6 Teile (53% der Theorie) eines Produktes der Formel

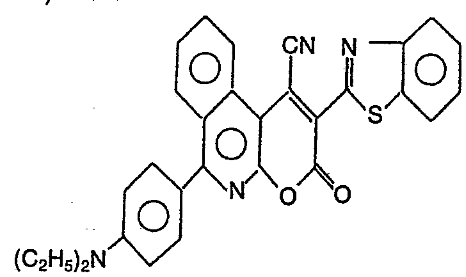

in Form schwarzer Kristalle vom Schmelzpunkt 247–249 °C, die Polyesterfasern in marineblauen Tönen färben.

Verfährt man analog den Beispielen 59 und 60, so erhält man weitere erfindungsgemässe Farbstoffe, die in der nachstehenden Tabelle aufgeführt sind.

Allgemeine Formel der Farbstoffe der nachstehenden Tabellenbeispiele 61–97

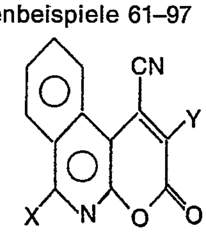

| Beispiel Nr. | X | Y | Farbton auf Polyesterfasern |
|---|---|---|---|
| 61 | 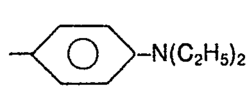 | 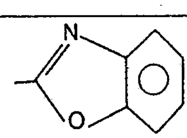 | blau |
| 62 | 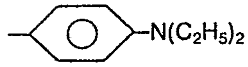 | 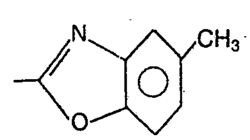 | blau |

| Beispiel Nr. | X | Y | Farbton auf Polyesterfasern |
|---|---|---|---|
| 63 | 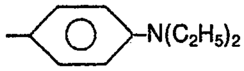 | 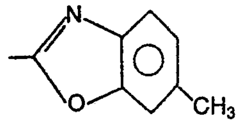 | blau |
| 64 | 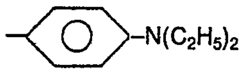 | 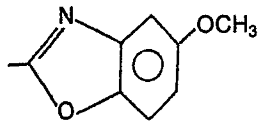 | blau |
| 65 | 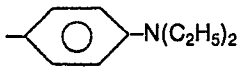 | 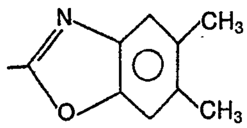 | blau |
| 66 | 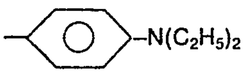 | 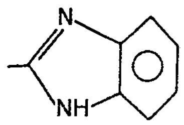 | blau |
| 67 | 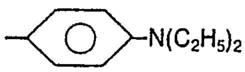 | 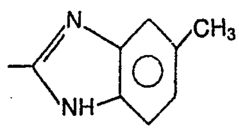 | blau |
| 68 | 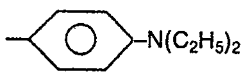 | 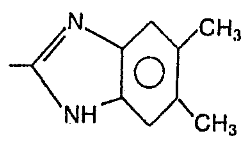 | blau |
| 69 | 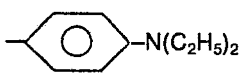 | 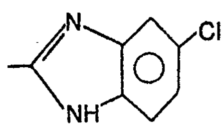 | blau |
| 70 | 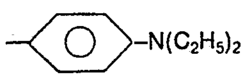 | 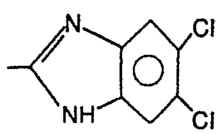 | blau |
| 71 | 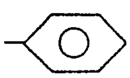 | 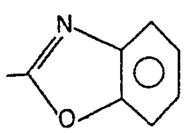 | rotstichiggelb |
| 72 | 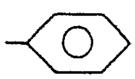 | 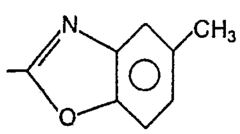 | rotstichiggelb |
| 73 | 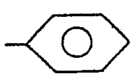 | 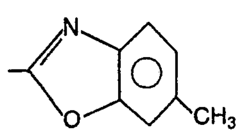 | rotstichiggelb |
| 74 | 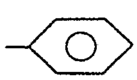 | 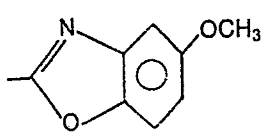 | rotstichiggelb |

| Beispiel Nr. | X | Y | Farbton auf Polyester-fasern |
|---|---|---|---|
| 75 | 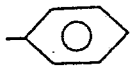 | 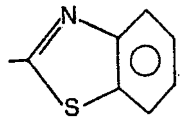 | rotstichiggelb |
| 76 | 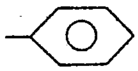 | 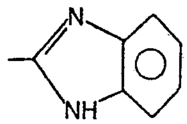 | rotstichiggelb |
| 77 | 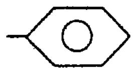 | 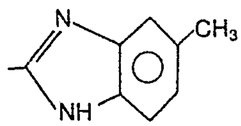 | rotstichiggelb |
| 78 | 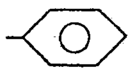 | 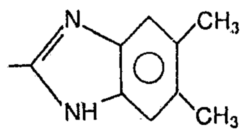 | rotstichiggelb |
| 79 | 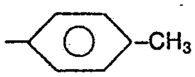 | 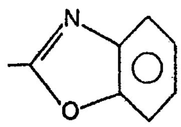 | rotstichiggelb |
| 80 | 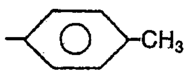 | 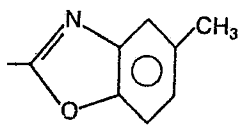 | rotstichiggelb |
| 81 | 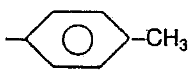 | 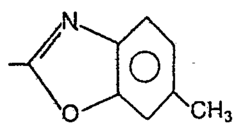 | rotstichiggelb |
| 82 | 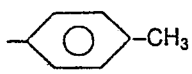 | 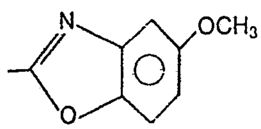 | rotstichiggelb |
| 83 | 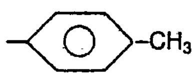 | 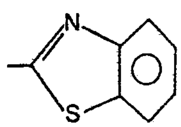 | rotstichiggelb |
| 84 | 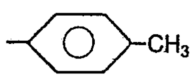 | 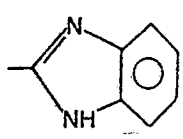 | rotstichiggelb |
| 85 | 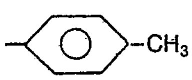 | 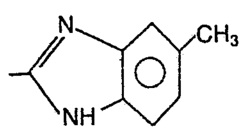 | rotstichiggelb |
| 86 | 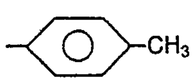 | 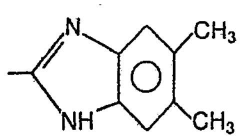 | rotstichiggelb |

| Beispiel Nr. | X | Y | Farbton auf Polyesterfasern |
|---|---|---|---|
| 87 | 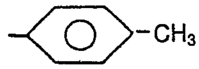 | 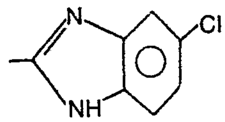 | rotstichiggelb |
| 88 | 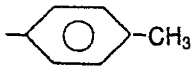 | 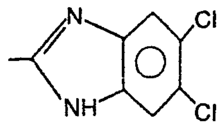 | rotstichiggelb |
| 89 | 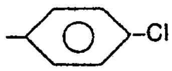 | 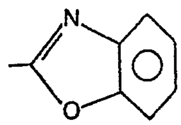 | rotstichiggelb |
| 90 | 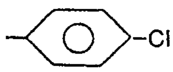 | 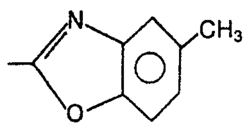 | rotstichiggelb |
| 91 | 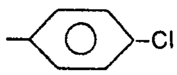 | 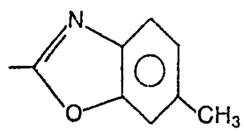 | rotstichiggelb |
| 92 | 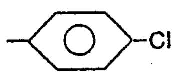 | 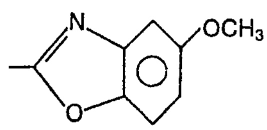 | rotstichiggelb |
| 93 | 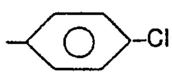 | 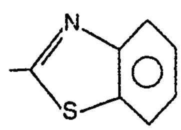 | rotstichiggelb |
| 94 | 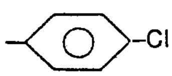 | 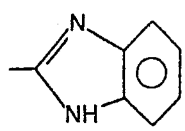 | rotstichiggelb |
| 95 | 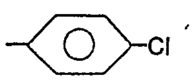 | 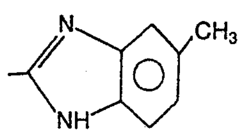 | rotstichiggelb |
| 96 | 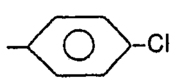 | 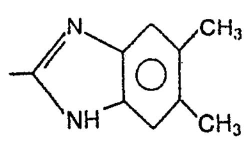 | rotstichiggelb |
| 97 | 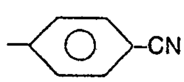 | 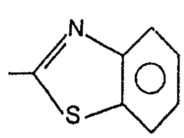 | rotstichiggelb |

Beispiel 98

4,4 Teile des nach Beispiel 59 hergestellten Farbstoffs werden mit 20 Teilen konz. Schwefelsäure 24 Stunden bei 25° C gerührt. Das ausgefallene Produkt wird abgesaugt, mit Wasser neutral gewaschen und bei 80° C im Vakuum getrocknet. Man erhält 3,2 Teile (69% der Theorie) orangegefärbtes Farbstoffpulver der Formel

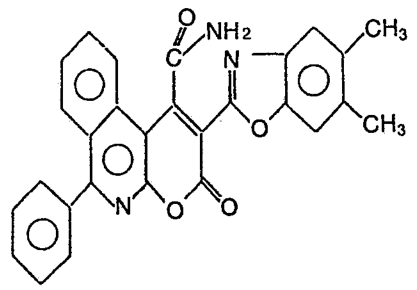

vom Schmelzpunkt 213–215° C.

**Patentansprüche für die Vertragsstaaten CH, DE, FR, GB, LI**

1. Azacumarine der allgemeinen Formel

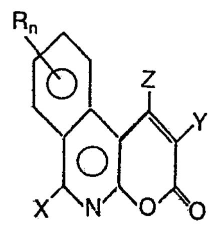

(1)

in welcher X einen ggfs. durch ein oder mehrere Fluor-, Chlor- oder Bromatome oder eine oder mehrere Alkyl$_{C_1-C_4}$, Alkoxy$_{C_1-C_4}$, Cyan-, Nitro-, Amino-, Monoalkyl$_{C_1-C_4}$amino-, Dialkyl$_{C_1-C_4}$amino-, Monoarylamino-, Diarylamino-, Carboxyl-, Carbonamid-, Carbonmonoalkyl$_{C_1-C_4}$amid-, Carbondialkyl$_{C_1-C_4}$amid-, Alkoxy$_{C_1C_4}$-carbonyl- oder Trifluormethylgruppen substituierten Phenylrest oder einen ggfs. durch Chlor- oder Bromatome oder Alkyl$_{C_1-C_4}$ oder Nitrogruppen substituierten Thiophen-, Pyridin-, Naphthalin- oder Pyrenrest bedeutet, Y ein Wasserstoffatom oder eine Cyan- oder Alkoxy$_{C_1-C_4}$carbonylgruppe, einen ggfs. durch Fluor-, Chlor-, oder Bromatome oder Alkyl$_{C_1-C_4}$, Alkoxy$_{C_1-C_4}$, Nitro-, Amino-, Monoalkyl$_{C_1-C_4}$amino- oder Dialkyl$_{C_1-C_4}$aminogruppen substituierten Phenylrest oder einen ggfs. durch Fluor-, Chlor- oder Bromatome oder Alkyl$_{C_1-C_4}$, Alkoxy$_{C_1-C_4}$, Nitro-, Cyan-, oder Carbonsäurealkyl$_{C_1-C_4}$estergruppen substituierten Thiophen-, Benzothiophen-, Furan-, Benzofuran-, Imidazol-, Benzimidazol-, Thiazol-, Benzthiazol-, Oxazol- oder Benzoxazolrest darstellt, Z ein Wasserstoffatom oder eine Carbonsäureamid- oder Cyangruppe bedeutet, R ein Wasserstoff-, Fluor-, Chlor- oder Bromatom oder eine Alkyl$_{C_1-C_4}$, Alkoxy$_{C_1-C_4}$, Nitro-, Amino-, Monoalkyl$_{C_1-C_4}$amino-, Dialkyl$_{C_1-C_4}$amino- oder Cyangruppe darstellt und n für die Zahl 1 oder 2 steht.

2. Azacumarin der Formel

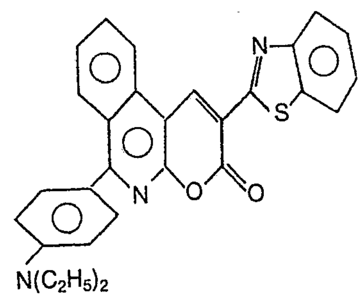

3. Azacumarin der Formel

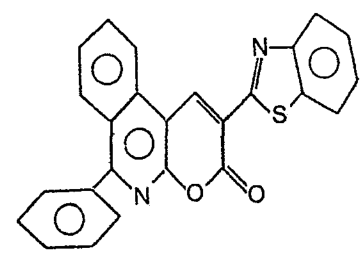

4. Azacumarin der Formel

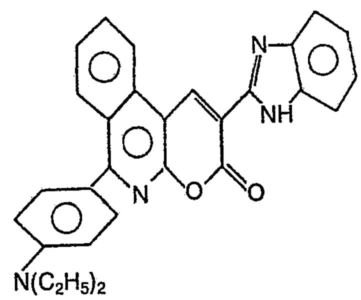

5. Azacumarin der Formel

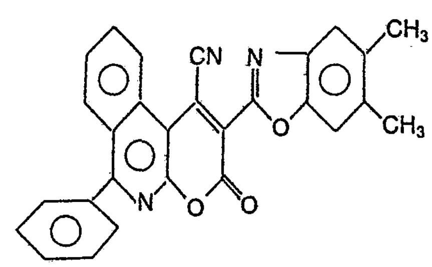

6. Azacumarin der Formel

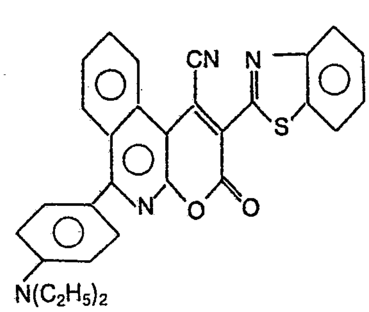

7. Azacumarin der Formel

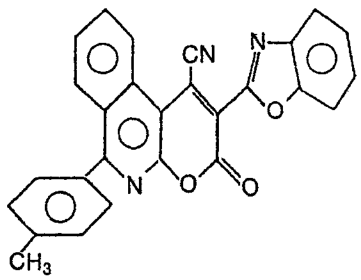

8. Verfahren zur Herstellung von Azacumarinen der in Anspruch 1 genannten allgemeinen Formel (1), dadurch gekennzeichnet, dass man eine Verbindung der allgemeinen Formel (2)

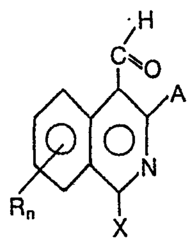

(2)

in welcher X, R und n die in Anspruch 1 genannten Bedeutungen haben, und A ein Chlor- oder Bromatom darstellt, mit einer Verbindung der allgemeinen Formel (3)

$$B - CH_2 - Y \qquad (3)$$

in welcher Y die in Anspruch 1 genannte Bedeutung hat, und B eine Cyano- oder Alkoxy$_{C_1-C_4}$carbonylgruppe darstellt, in an sich bekannter Weise kondensiert, die hierbei erhaltene Verbindung der allgemeinen Formel (4)

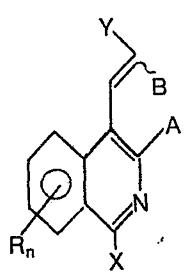

(4)

in welcher A, B, X, Y, R und n die angegebenen Bedeutungen haben, mit einer nichtoxidierenden wässrigen Mineralsäure bei Temperaturen von 80 bis 140 °C cyclisiert zu einer Verbindung der genannten allgemeinen Formel (1) mit Z = H und diese ggfs. nach an sich bekannten Verfahren mit einem Cyanidsalz umsetzt und die hierbei als Zwischenprodukt gebildete Anlagerungsverbindung gleichzeitig oder anschliessend nach an sich bekannten Verfahren mit einem Oxydationsmittel behandelt unter Erhalt der Verbindung der allgemeinen Formel (1) mit Z = CN und diese ggfs. in an sich bekannter Weise verseift zur Verbindung der allgemeinen Formel (1) mit Z = CO–$NH_2$.

9. Verwendung der Azacumarine der in Anspruch 1 genannten allgemeinen Formel (1) als Farbstoffe zum Färben oder Bedrucken von synthetischen Fasermaterialien, insbesondere solchen aus linearen Polyestern, ggfs. in Mischung mit natürlichen Fasermaterialien.

10. Verwendung nach Anspruch 9, dadurch gekennzeichnet, dass die Farbstoffe zum Färben oder Bedrucken von Fasermaterialien aus Cellulose-di-, -2$\frac{1}{2}$- oder -triacetat, Polyamiden, Polyurethanen, Polycarbonaten oder Polyäthylenterephthalat, ggfs. in Mischung mit Baumwolle oder Wolle eingesetzt werden.

11. Verwendung der Azacumarine der in Anspruch 1 genannten allgemeinen Formel (1) als Farbstoffe zum Schmelzspinnfärben von Polyestern.

12. Verwendung der Azacumarine der in Anspruch 1 genannten allgemeinen Formel (1) als Farbstoffe zum Färben von Kunststoffen in der Masse.

13. Verwendung der Azacumarine der in Anspruch 1 genannten allgemeinen Formel (1) in Lichtsammelsystemen.

**Claims for the Contracting States CH, DE, FR, GB, LI**

1. An azacoumarin of the general formula

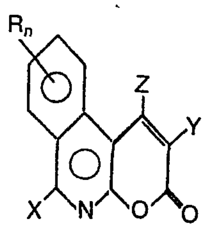

(1)

in which X denotes a phenyl radical which is optionally substituted by one or more fluorine, chlorine or bromine atoms or one or more alkyl$_{C_1-C_4}$, alkoxy$_{C_1-C_4}$, cyano, nitro, amino, monoalkyl$_{C_1-C_4}$-amino, dialkyl$_{C_1-C_4}$-amino, monoarylamino, diarylamino, carboxyl, carboxamide, monoalkyl$_{C_1-C_4}$carboxamide, dialkyl$_{C_1-C_4}$carboxamide, alkoxy$_{C_1-C_4}$carbonyl or trifluoromethyl groups or a thiophene, pyridine, naphthalene or pyrene radical which is optionally substituted by chlorine or bromine atoms or alkyl$_{C_1-C_4}$ or nitro groups, Y represents a hydrogen atom or a cyano or alkoxy$_{C_1-C_4}$-carbonyl group, a phenyl radical which is optionally substituted by fluorine, chlorine or bromine atoms or alkyl$_{C_1-C_4}$, alkoxy$_{C_1-C_4}$, nitro, amino, monoalkyl$_{C_1-C_4}$amino or dialkyl$_{C_1-C_4}$amino groups or a thiophene, benzothiophene, furan, benzofuran, imidazole, benzimidazole, thiazole, benzothiazole, oxazole or benzoxazole radical which is optionally substituted by fluorine, chlorine or bromine atoms or alkyl$_{C_1-C_4}$, alkoxy$_{C_1-C_4}$, nitro, cyano or alkyl$_{C_1-C_4}$ carboxylate groups, Z denotes a hydrogen atom or a carboxamide or cyano

group, R represents a hydrogen, fluorine, chlorine or bromine atom or an alkyl$_{C_1-C_4}$, alkoxy$_{C_1-C_4}$, nitro, amino, monoalkyl$_{C_1-C_4}$amino, dialkyl$_{C_1-C_4}$amino or cyano group, and n represents the number 1 or 2.

2. The azacoumarin of the formula

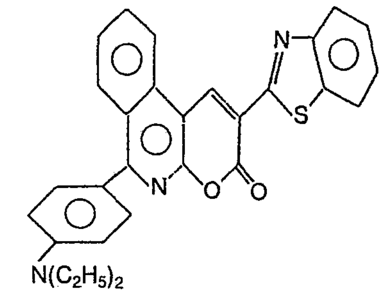

3. The azacoumarin of the formula

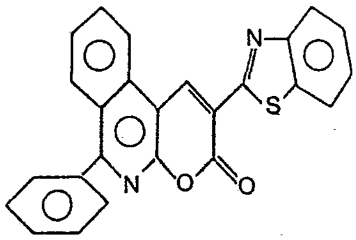

4. The azacoumarin of the formula

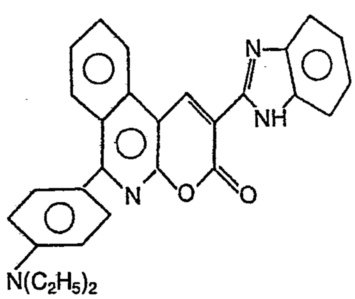

5. The azacoumarin of the formula

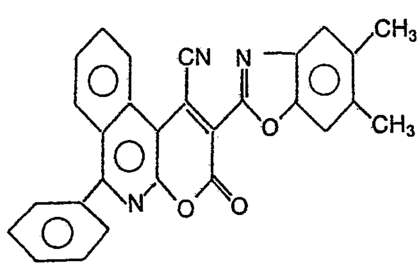

6. The azacoumarin of the formula

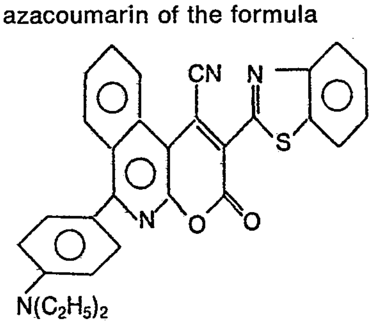

7. The azacoumarin of the formula

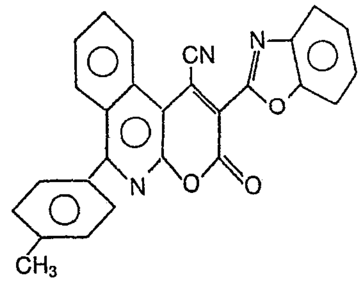

8. A process for preparing an azacoumarin of the general formula (1) mentioned in claim 1, which comprises condensing a compound of the general formula (2)

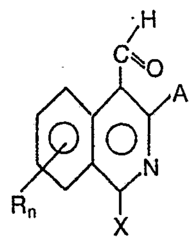

(2)

in which X, R and n have the meanings mentioned in claim 1, and A represents a chlorine or bromine atom, with a compound of the general formula (3)

$$B - CH_2 - Y \qquad (3)$$

in which Y has the meaning mentioned in claim 1, and B represents a cyano or alkoxy$_{C_1-C_4}$carbonyl group, in a manner known per se, cyclizing the resulting compound of the general formula (4)

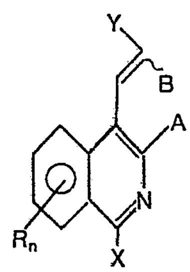

in which A, B, X, Y, R and n have the abovementioned meanings, with a non-oxidizing aqueous mineral acid at temperatures of 80 to 140 °C to give a compound of the said general formula (1) in which Z = H, optionally reacting this compound with a cyanide salt by methods known per se, simultaneously or subsequently treating the addition compound formed as the intermediate product by methods known per se with an oxidizing agent to give the compound of the general formula (1) in which Z = CN, and optionally hydrolyzing this compound in a manner known per se to give the compound of the general formula (1) which Z = CO–$NH_2$.

9. The use of an azacoumarin of the general formula (1), mentioned in claim 1, as a dyestuff for dyeing or printing synthetic fiber materials, in particular those made of linear polyesters, if desired in a blend with natural fiber materials.

10. The use as claimed in claim 9, wherein the dyestuff is used for dyeing or printing fiber materials made of diacetate, secondary acetate or triacetate rayon, polyamides, polyurethanes, polycarbonates or polyethylene terephthalate, if desired in a blend with cotton or wool.

11. The use of an azacoumarin of the general formula (1), mentioned in claim 1, as a dyestuff for spin-dyeing polyester melts.

12. The use of an azacoumarin of the general formula (1), mentioned in claim 1, as a dyestuff for mass-coloring plastics.

13. The use of an azacoumarin of the formula (1), mentioned in claim 1, in light-collecting systems.

**Revendications pour les Etats Contractants CH, DE, FR, GB, LI**

1. Azacoumarines répondant à la formule générale 1:

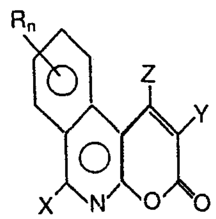

(1)

dans laquelle:

X représente un radical phényle éventuellement porteur d'un ou de plusieurs atomes de fluor, de chlore ou de brome ou d'un ou de plusieurs radicaux alkyles$_{C_1-C_4}$, alcoxy$_{C_1-C_4}$, cyano, nitro, amino, monoalkyl$_{C_1-C_4}$amino, dialkyl$_{C_1-C_4}$amino, monoarylamino, diarylamino, carboxy, carbamoyle, N-monoalkyl$_{C_1-C_4}$carbamoyle, N,N-dialkyl$_{C_1-C_4}$carbamoyle, alcoxy$_{C_1-C_4}$carbonyle ou trifluorométhyles, ou un radical de thiophène, de pyridine, de naphtalène ou de pyrène éventuellement porteur, par exemple, d'atomes de chlore ou de brome ou de radicaux alkyles$_{C_1-C_4}$ ou nitro, Y représente un atome d'hydrogène, un radical cyano, un radical alcoxy$_{C_1-C_4}$carbonyle, un radical phényle éventuellement porteur, d'atomes de fluor, de chlore ou de brome ou de radicaux alkyles$_{C_1-C_4}$, alcoxy$_{C_1-C_4}$, nitro, amino, monoalkyl$_{C_1-C_4}$amino ou dialkyl$_{C_1-C_4}$amino, ou un radical de thiophène, de benzothiophène, de furanne, de benzofuranne, d'imidazole, de benzimidazole, de thiazole, de benzothiazole, d'oxazole ou de benzoxazole éventuellement porteur, d'atomes de fluor, de chlore ou de brome ou de radicaux alkyles$_{C_1-C_4}$, alcoxy$_{C_1-C_4}$, nitro, cyano ou alcoxy$_{C_1-C_4}$carbonyles, Z représente un atome d'hydrogène ou un radical carbamoyle ou cyano, R représente un atome d'hydrogène, de fluor, de chlore ou de brome ou un radical alkyle$_{C_1-C_4}$, alcoxy$_{C_1-C_4}$, nitro, amino, monoalkyl$_{C_1-C_4}$amino, dialkyl$_{C_1-C_4}$amino ou cyano, et n représente le nombre 1 ou le nombre 2.

2. Azacoumarine de formule:

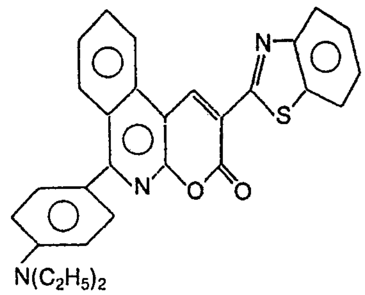

3. Azacoumarine de formule:

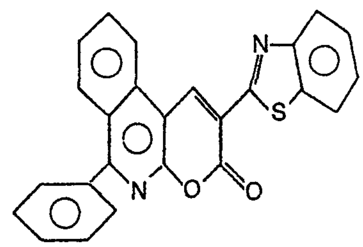

4. Azacoumarine de formule:

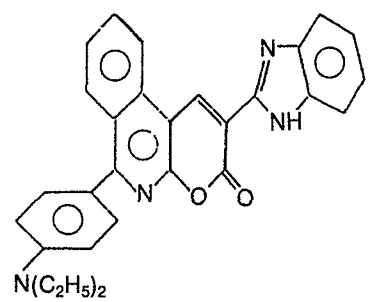

5. Azacoumarine de formule:

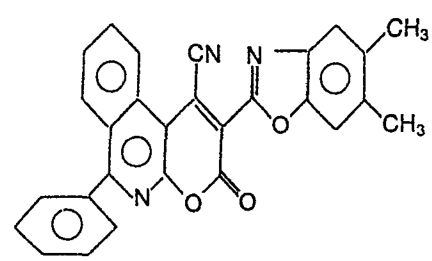

6. Azacoumarine de formule:

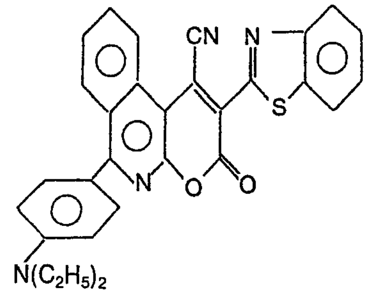

7. Azacoumarine de formule:

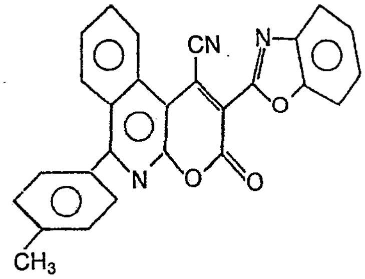

8. Procédé de préparation d'azacoumarines de formule générale 1 selon la revendication 1, procédé caractérisé en ce qu'on condense de manière connue un composé répondant à la formule générale 2:

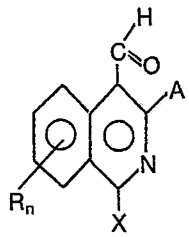

(2)

dans laquelle X, R et n ont les significations données à la revendication 1 et A représente un atome de chlore ou de brome, avec un composé répondant à la formule générale 3:

$$B - CH_2 - Y \quad (3)$$

dans laquelle Y a la signification donnée à la revendication 1 et B représente un radical cyano ou alcoxy$_{\overline{C_1-C_4}}$carbonyle, on cyclise le composé ainsi obtenu qui répond à la formule générale 4:

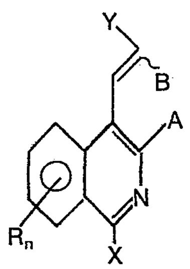

dans laquelle A, B, X, Y, R et n ont les significations précédemment données, au moyen d'un acide minéral aqueux non oxydant, à des températures de 80 à 140 °C, cyclisation qui conduit à un composé de formule générale 1 dans lequel Z représente H, et on fait réagir éventuellement ce dernier, par des méthodes connues, avec un sel de l'acide cyanhydrique, on traite de manière connue le composé d'addition qui s'est alors formé comme corps intermédiaire, en même temps ou ultérieurement, par un agent d'oxydation, de manière à obtenir le composé de formule générale 1 dans lequel Z représente $-CN$, et on saponifie éventuellement celui-ci, de manière connue, pour le convertir en le composé de formule générale 1 dans lequel Z représente $-CO-NH_2$.

9. Application des azacoumarines de formule générale 1 selon la revendication 1 comme colorants pour la teinture ou pour l'impression de matières fibreuses synthétiques, plus particulièrement de matières fibreuses en polyesters linéaires, éventuellement en mélange avec des matières fibreuses naturelles.

10. Application selon la revendication 9 caractérisée en ce que les colorants sont utilisés pour la teinture ou pour l'impression de matières fibreuses en diacétate, en hémipenta-acétate ou en triacétate de cellulose, en polyamides, en polyuréthannes, en polycarbonates ou en poly-(téréphtalate d'éthylène), éventuellement en mélange avec du coton ou de la laine.

11. Application des azacoumarines de formule générale 1 selon la revendication 1 comme colorants pour la teinture au filage à l'état fondu de polyesters.

12. Application des azacoumarines de formule générale 1 selon la revendication 1 comme colorants pour la teinture de matières plastiques dans la masse.

13. Application des azacoumarines de formule générale 1 selon la revendication 1 dans des systèmes collecteurs de lumière.